(19) **Europäisches Patentamt / European Patent Office / Office européen des brevets**

(11) **EP 4 322 847 B1**

(12) **EUROPEAN PATENT SPECIFICATION**

(45) Date of publication and mention
of the grant of the patent:
**12.03.2025  Bulletin 2025/11**

(21) Application number: **22715899.5**

(22) Date of filing: **22.03.2022**

(51) International Patent Classification (IPC):
**G06F 21/44** (2013.01)    **A61B 5/07** (2006.01)
**G16H 40/67** (2018.01)    **A61B 5/00** (2006.01)
**G16H 20/10** (2018.01)

(52) Cooperative Patent Classification (CPC):
**G06F 21/44; A61B 5/073; A61B 5/4833;
A61B 5/6861; G16H 20/10; G16H 40/67;**
A61B 2562/08

(86) International application number:
**PCT/US2022/021418**

(87) International publication number:
**WO 2022/221007 (20.10.2022 Gazette 2022/42)**

(54) **MULTIPLE MEDICATION INGESTION DETECTION**

MEHRFACHDETEKTION VON ARZNEIMITTELAUFNAHME

DÉTECTION DE L'INGESTION DE PLUSIEURS MÉDICAMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **14.04.2021   US 202163174896 P**

(43) Date of publication of application:
**21.02.2024   Bulletin 2024/08**

(73) Proprietor: **Etectrx, Inc.**
**Gainesville, FL 32601 (US)**

(72) Inventors:
• **MYERS, Brent Arnold**
 **Palm Bay, FL 32907 (US)**
• **FROGGE, Perry**
 **Palm Bay, FL 32907 (US)**
• **BELL, Timothy**
 **Indian Harbour Beach, FL 32937 (US)**
• **BUFFKIN, Eric**
 **Newberry, FL 32669 (US)**

(74) Representative: **Bardehle Pagenberg
Partnerschaft mbB
Patentanwälte Rechtsanwälte
Prinzregentenplatz 7
81675 München (DE)**

(56) References cited:
 **US-A1- 2017 258 362     US-B2- 8 115 618**

• **BASAR M. R. ET AL: "Ingestible Wireless
Capsule Technology: A Review of Development
and Future Indication", INTERNATIONAL
JOURNAL OF ANTENNAS AND PROPAGATION,
vol. 2012, no. 14, 1 January 2012 (2012-01-01),
pages 1 - 14, XP055935222, ISSN: 1687-5869,
Retrieved from the Internet <URL:https://
downloads.hindawi.com/journals/ijap/2012/
807165.pdf> DOI: 10.1155/2012/807165**

**Description**

**Cross-Reference to Related Applications**

This application claims priority to and the benefit of U.S. Provisional Application No. 63/174,896, filed on April 14, 2021, entitled "Multiple Medication Ingestion Detection".

**Field of Technology**

This disclosure generally relates to medication ingestion detection and, more particularly, to a method and system for identifying multiple capsule ingestions within the same time period.

**Background**

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Non-compliance of patients with drug regimens prescribed by physicians can cause a multiplicity of problems, including negative patient outcomes, higher healthcare costs and an increased risk of the spread of communicable diseases. Other areas where compliance can be critical is in, for example, pharmaceutical clinical trials, geriatrics and mental health/addition medicine. It is beneficial, then, to provide compliance monitoring. Compliance monitoring can take the form of direct observance or in vivo biotelemetry and monitoring.

US 2017/258362 A1 relates to an ingestible bio-telemetry communication network and associated systems. The communication network can include one or more ingestible bio-telemetry tags; and a reader, wherein each of the one or more ingestible bio-telemetry tags generates an out-link signal comprising, for each bit of data in a frame, a pulse reverse keyed symbol. Multiple ingestible bio-telemetry tags can be managed at the same time by allowing the frequency of the transmit carrier signal to change, or "hop" to different frequencies so as to minimize likelihood of collision. A reader can identify the proper frequency either by a signal from the tags indicated the frequency of the next hop or, when no bi-directional communication is available, by deducing the carrier signal frequency from the start bits of a received frame from the tag and scanning for the shifted carrier signal frequency within a tolerance of the deduced carrier signal frequency.

**Summary**

Compliance monitoring may be performed with ingestible adherence sensors which transmit data from a patient's gastrointestinal (GI) tract indicating a patient has taken medication properly. Each ingestible adherence sensor may be coupled to a medication capsule. In some implementations, each of the capsules is for the same medication (e.g., three aspirin capsules), or may be for different medications. To identify multiple capsules ingested within the same time period (e.g., 20 minutes), a capsule detection system detects characteristics of the signals transmitted by the ingestible adherence sensors. Additionally, the capsule detection system may decode a message included in a signal from an ingestible adherence sensor, where the message includes message fields such as a serial number field, a battery level field, a medication identifier field, etc.

Each ingestible adherence sensor converts chemical energy activated by the patient's stomach fluid to electromagnetic energy having a frequency of about 300MHz. More specifically, the ingestible adherence sensor may modulate a 300MHz carrier wave using a series of pulses having a pulse width of about 1-4 $\mu$s and a pulse period or spacing of about 1-1.5 ms. In some implementations, the capsule detection system identifies multiple capsules based on differences in the characteristics of the pulses, such as differences in frequency or differences in pulse spacing. For example, when the pulse spacing from two signals or transmissions differs by more than a threshold amount (e.g., 6 $\mu$s), the capsule detection system may identify the two transmissions as corresponding to two different capsules. However, the pulse spacing of these signals appears to cluster over a relatively small range (e.g., 42 $\mu$s). Accordingly, two or more capsules may transmit signals with pulse spacing that does not differ by more than the threshold amount, and the transmissions may be incorrectly identified as corresponding to the same capsule.

Furthermore, the pulse spacing of transmissions from an ingestible adherence sensor may vary over time based on the battery level for the ingestible adherence sensor. As the battery level decreases, the pulse space between pulses may increase.

To enable the detection of multiple capsules ingested within the same time period (e.g., 20 minutes), the ingestible adherence sensor transmits an indication of the battery level of the battery and an indication of a serial number in a transmission having a particular pulse space to a receiver. The serial number may be a randomly generated number

**EP 4 322 847 B1**

which may be used to distinguish between transmissions from different capsules. In some implementations, the serial number is generated based on properties of electrical components within a serial number generation circuit. For example, the electrical components may affect the output voltage of the serial number generation circuit and the serial number may be a digitized value selected based on the output voltage. More specifically, the serial number generation circuit may include electrical components with variations in their component values (e.g., a 10 kΩ resistor with a 10 percent tolerance), such that the actual component values of the electrical components differ from their expected component values. The variation in the actual component values causes the serial numbers to be selected randomly. While the actual component values may differ from the expected component values, the actual component values do not change over time. In this manner, a serial number may be selected randomly, but once selected, the random serial number will stay the same even if the ingestible adherence sensor resets, for example due to battery issues.

The receiver then compares the battery level, the pulse space, and/or the serial number for the transmission to battery levels, pulse spaces, and/or serial numbers for other transmissions to determine the number of capsules ingested. More specifically, the receiver may detect two transmissions at a time within a detection period (e.g. 180 ms) using two detectors. For example, a first detector may detect a first pulse, may ignore subsequent pulses that occur before a threshold pulse spacing time window (e.g., 1.2-1.4 ms), and may identify additional pulses occurring within respective threshold pulse spacing time windows to identify a first transmission. The receiver may then generate a blanking mask which filters the pulses detected at the first detector from being detected at the second detector. The second detector then detects a second pulse which was ignored by the first detector, may ignore subsequent pulses that occur before a threshold pulse spacing time window (e.g., 1.2-1.4 ms), and may identify additional pulses occurring within respective threshold pulse spacing time windows to identify a second transmission. Then the receiver compares the battery levels, pulse spaces, and/or serial numbers of the first and second transmissions to each other and/or to transmissions detected during previous detection periods to determine whether either or both of the transmissions correspond to additional capsules. Each time an additional capsule is detected, the receiver increments the count of the number of capsules and stores the serial number and pulse spacing information for the identified capsule. The stored serial numbers and pulse spacing information are then compared to subsequent serial numbers and pulse spacing information detected during subsequent detection periods.

One example embodiment of the techniques of this disclosure is a method for enabling detection of a plurality of capsule ingestions. For each of a plurality of ingestible capsules including a transmitter and processing hardware to generate and transmit signals, the method includes obtaining a serial number for distinguishing between the plurality of ingestible capsules, and generating a signal indicating that the capsule has been ingested. The signal includes a series of pulses having a particular pulse space and indicates the serial number. The method also includes transmitting the signal to a receiver via the transmitter. Each of the plurality of ingestible capsules is identified based on at least one of: the particular pulse space and the serial number for the ingestible capsule.

Another example embodiment of the techniques of this disclosure is a method for identifying a plurality of capsule ingestions. The method includes receiving, from a first ingestible capsule, a first transmission having a first series of pulses with a first pulse space and including an indication of a first serial number, and receiving, from a second ingestible capsule, a second transmission having a second series of pulses with a second pulse space and including an indication of a second serial number. The method further includes identifying the first and second transmissions as corresponding to at least two ingestible capsules based on at least one of: (i) the first and second pulse spaces, and (ii) the first and second serial numbers.

Yet another example embodiment of the techniques of this disclosure is an ingestible bio-telemetry tag system. The system includes a capsule, a transmitter, and processing hardware coupled to the capsule and the transmitter. The processing hardware includes an electrical component with an actual component value that varies amongst a plurality of electrical components having a same expected component value as the electrical component. The actual component value does not vary over time. The processing hardware is configured to generate an output voltage in accordance with the actual component value for the electrical component, and generate a random serial number based on the output voltage. The random serial number remains the same each time the ingestible bio-telemetry tag system is reset

**Brief Description of the Drawings**

Fig. 1 is a block diagram of an example capsule detection system in which the techniques of this disclosure can be implemented;

Fig. 2 illustrates an example signal transmitted by an ingestible adherence sensor including a series of pulses having a particular pulse space;

3

Fig. 3 illustrates an example graph depicting a distribution of pulse spaces in transmissions from several ingestible adherence sensors;

Figs. 4A-4B illustrate example graphs depicting the battery level in an ingestible adherence sensor and pulse spacing of pulses transmitted by the ingestible adherence sensor, respectively, over time;

Fig. 5 illustrates an example circuit diagram of an ingestible adherence sensor to enable the detection of multiple capsules;

Fig. 6 illustrates an example circuit diagram of a serial number generation circuit for generating a serial number to include in a transmission by an ingestible adherence sensor, which may be a subcircuit of the example circuit illustrated in Fig. 5;

Fig. 7 illustrates an example message having battery level, serial number, and other message fields which may be transmitted by the ingestible adherence sensor;

Figs. 8A-8B illustrate example block diagrams of a receiver that receives transmissions from multiple ingestible adherence sensors;

Fig. 9 illustrates a block diagram of an example dual detection system within a receiver that includes two detectors for simultaneously detecting two transmissions;

Figs. 10A-10B illustrate example timing diagrams depicting pulses from two different capsules detected at the same time and separated out to show the pulses from the first capsule in one timing diagram and the pulses from the second capsule in another timing diagram;

Fig. 11A illustrates an example graph depicting pulse spacing of pulses transmitted by four ingestible adherence sensors over time;

Fig. 11B illustrates an example graph depicting adjusted pulse spacing of pulses transmitted by the four ingestible adherence sensors in Fig. 11A over time, where the pulse spaces are adjusted in accordance with the battery levels of the respective ingestible adherence adherences;

Fig. 12 illustrates an example graph depicting the accuracy of the multiple capsule detection as a function of the number of possible serial number values which may be included in transmissions by the ingestible adherence sensors;

Fig. 13 illustrates another example graph depicting the accuracy of the multiple capsule detection as a function of the number of capsules and the number of possible serial number values which may be included in transmissions by the ingestible adherence sensors;

Fig. 14 illustrates a flow diagram of an example method for enabling detection of a plurality of capsule ingestions, which may be implemented by an ingestible adherence sensor; and

Fig. 15 illustrates a flow diagram of an example method for identifying a plurality of capsule ingestions, which may be implemented by an example receiver.

**Detailed Description**

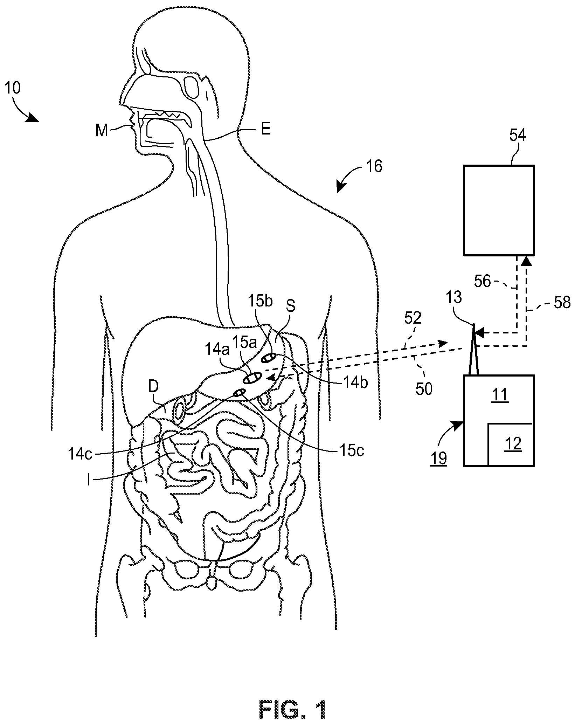

Fig. 1 illustrates an example capsule detection system 10 for detecting multiple capsules ingested over the same time period. Referring to Fig. 1, the system 10 for monitoring medication compliance in a patient 16 includes an electronic sensor 11, preferably in the form of an external wireless monitor or reader 11 (also referred to herein as a "receiver") that includes a (radiofrequency) RF transceiver 12 and one or more antennas 13. The antenna 13 can be external or internal to the reader 11 and can be implemented in a variety of ways as known in the art, including an on-chip antenna or simple pads or electrical contacts that function as an antenna. The reader 11 detects the presence of electronic pills 14a-14c (also referred to herein as an "ingestible bio-telemetry tag system") in, for example, the gastrointestinal (GI) tract of the patient 16. As shown, the electronic pills 14a-14c have ingestible adherence sensors or TAGs 15a-15c that are attached to or part of the pills 14a-14c. For purposes of this disclosure, the term "pill" can include a capsule or other form of medication administration or testing. The system 10 is designed to detect the pills 14a-14c when located in the patient's 16 mouth M, esophagus E, stomach S, duodenum D, intestines (including the colon) I, or rectum R.

As mentioned above, the system 10 includes a TAG 15a-15c fixed with each pill 14a-14c, either internally or along the outer surface, or both. After ingestion of the pill 14a-14c, the TAG 15a-15c can become or be made electronically active and begins communication with the external reader 11. More specifically, the TAG 15a-15c includes an electrochemical battery activated by the patient's 16 stomach fluid. The external reader 11 may be in a housing 19 worn by or attached with the patient 16 so as to be comfortable and easy to wear continuously to ensure it is always with the patient.

The electronic pill 14a-14c comprises an orally ingestible and biocompatible drug-transporting device with embedded or attached electronic circuits (the TAG 15a-15c) that communicate with the external wireless reader 11. The electronic pill 14a-14c, and more particularly the TAG 15a-15c, has, for example, a silicon-based integrated circuit and/or other passive components such as coil antennae and capacitors. The circuit can incorporate millions of transistors, patterned through various semiconductor processing steps, to provide an enormous amount of intelligence. For instance, the electronic pill 14a-14c can store a patient's medical history in addition to detailed information about a drug being administered, provide a unique identification number, and implement advanced communication circuits and protocols to reliably transmit data to the external wireless reader 11.

In use, one or more electronic pills 14a-14c may be taken by a patient 16. The data reader 11 and the one or more TAGs 15a-15c can exchange bidirectional data 50/52. The reader 11 may probe the one or more TAGs 15a-15c inside the patient 16 and coordinates, when possible, the communication between the possibly multiple TAGs 15a-15c and the reader 11. Multiple ingested tags may communicate simultaneously, sequentially, or in other ways.

The TAGs 15a-15c communicate their unique identification data and, in some cases, whether they are in the GI tract. The reader 11 can provide output data 58 to a user interface 54 such as a laptop or smartphone enabling, in some implementations, real-time upload of medication events to a remote database or other location. The reader 11 may receive, via the channel 56, information from the user interface 54 indicating medication regimen status such as the time of the next scheduled medication event, confirmation of the event from the main database, or other information from the user interface 54 or the remote database or trial coordination center via a wide area network (cell or Wi-Fi network) channel. In some cases, the user interface 54 and reader 11 are embedded into a single device, either on or off the body.

The data link from the reader 11 to a TAG 15a-15c is defined as the "in-link" path 50. In-link data to the TAG may include, but not be limited to, at least one of synchronization, signaling, address, and tag configuration information. The reader 11 may transmit information by way of differential metallic skin contacts. The in-link signal 50 passes through the body of patient 16 and can be sensed by the TAG 15a-15c through a differential probe network.

The data link from the TAG 15a-15c to the reader 11 is defined as the "out-link" path 52. Out-link data to the reader may include, but not be limited to, at least one of GI sensing, battery level information, a serial number, pharmaceutical, adherence, signal level, physiologic data, biometric identification data, and address information. The out-link channel 52 is a radio frequency signal traveling through both the body of the patient 16 and the free space between the body and the antenna of the reader 11. A small antenna on the TAG 15a-15c radiates the out-link signal 52 which is received at the reader 11. The reader 11 can be capable of receiving signals 52 from multiple TAGs 15a-15c simultaneously. System 10 - with TAGs 15a-15c and reader 11 work together to complete a system that can accurately detect a medication event, including the time of ingestion, the dosage, specific identification of the medication, the number of capsules ingested, and/or the subject using the system. This information is then used to verify critical compliance with drug therapy. This data can also be used in combination with other patient data to improve adherence and treatment outcomes.

As illustrated in FIG. 1 and described above, a capsule detection system 10 can be a bi-directional telemetry system providing an in-link signal and an out-link signal. An in-link signal is a synchronizing signal that is transmitted from a patch or other injection mechanism to an ingested TAG 15a-15c. An out-link signal is generated by the TAG 15a-15c by using the received synchronizing signal. The out-link signal is a frequency stable, outbound telemetry signal that incorporates the desired telemetry data (medication type, dosage, serial number, etc.).

The out-link signal can be a series of pulse modulated radio frequency (RF) signals having a duration of typically 1 to 4 $\mu$s and a pulse period or pulse space of between 1 and 1.5 ms. This is about a 1 to 1000 duty cycle which means the signal is absent far more than it is present. The pulses are present based on a modulation scheme. For example, in a pulse amplitude modulation scheme, the presence of a pulse may represent a transmitted data bit of one while the absence of a pulse may represent a transmitted data bit of zero. To improve the signal to noise ratio, the pulses may be arranged such that N pulses (e.g., five pulses) represent data one, while the lack of N consecutive pulses may represent a zero.

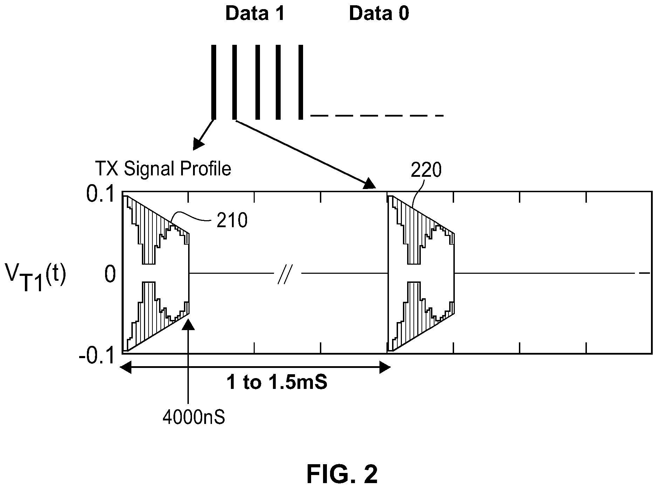

For example, as shown in Fig. 2, each pulse 210, 220 has a maximum and minimum voltage of about 0.1V and -0.1V, and a duration of about 4 $\mu$s. The period between consecutive pulses 210, 220 or pulse spacing is about 1-1.5 ms, and five consecutive pulses indicates a data one while five consecutive pulse periods without a pulse indicates a data zero.

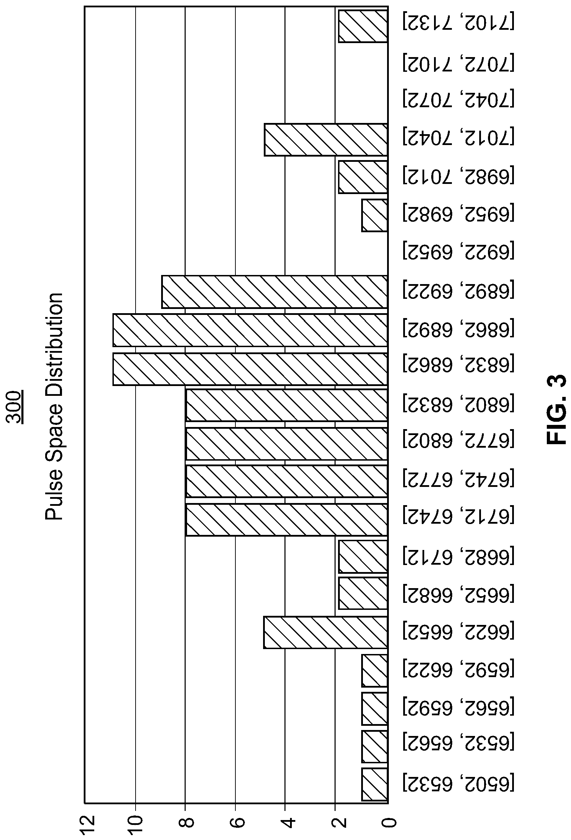

As mentioned above, one way to detect multiple capsules may be to identify differences in pulse spacing from signals emitted by the TAGs 15a-15c. Fig. 3 illustrates an example graph 300 depicting a distribution of pulse spaces in transmissions from several ingestible adherence sensors or TAGs 15a-15c. In the graph 300, the pulse spaces are converted from time periods to samples based on a sample period of 0.2 $\mu$s. The number of samples is equal to the pulse space divided by the sample period (e.g., 0.2 μs). For example, a transmission having a pulse space of 1320 μs has 6600 samples. Additionally, in the graph 300 each of the pulse space values is assigned to a bin having a sample range (e.g., between 6772 and 6802 samples) spanning 30 samples or about 6 μs. The graph 300 is a histogram of the number of transmissions from TAGs having pulse spaces assigned to each bin. For example, there are about 11 transmissions having pulse space values between 6832 and 6862 samples. There are about eight transmissions having pulse space values between 6712 and 6742 samples. As shown in Fig. 3, the majority of the transmissions have pulse spaces within seven bins. As a result, it is likely that two or more transmissions from two or more TAGs 15a-15c have pulse spaces within the same bin.

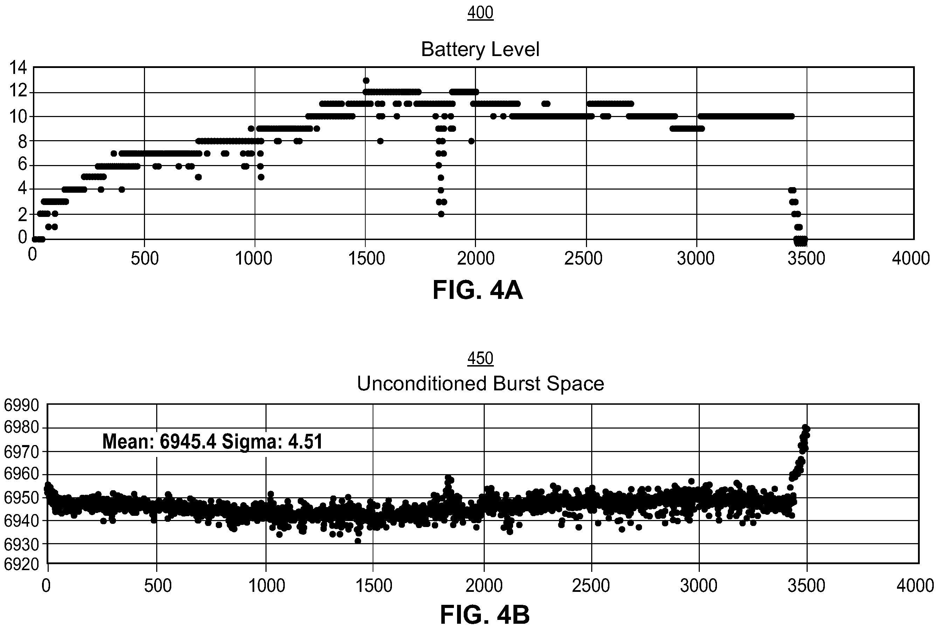

Additionally, the pulse space value for transmissions from the same TAG 15a-15c may change over time based on the battery level of the TAG 15a-15c. Fig. 4A illustrates an example graph 400 depicting the battery level of a TAG 15a-15c over the lifetime of the capsule 14a-14c (e.g., 20 minutes) in the patient's 16 stomach. Fig. 4B illustrates an example graph 450 depicting the pulse space of transmissions by the same TAG 15a-15c over the lifetime of the capsule 14a-14c (e.g., 20 minutes) in the patient's 16 stomach. As shown in Figs. 4A and 4B, as the battery level increases, the pulse space decreases. Then when the battery level decreases, the pulse space increases. At the end of the lifetime of the capsule 14a-14c, the battery level decreases, and the pulse space increases significantly. Accordingly, the reader 11 adjusts the pulse space based on the current battery level so that a change in the pulse space does not cause the reader 11 to identify the transmissions from the TAG 15a-15c as multiple transmissions from multiple TAGs 15a-15c. For example, the reader 11 may discard the pulse space when the battery level is below a threshold voltage.

To enable the reader 11 to detect multiple capsules ingested over the same time period (e.g., 20 minutes), the TAG 15a-15c includes processing hardware configured to generate a battery level and a serial number. The processing hardware may then generate a transmission including a battery level field and a serial number field with indications of the respective battery level and serial number. The serial number may be a randomly generated number which may be one, two, three, four, or five bits. In other implementations, the serial number may be a number larger than 5 bits, but less than a maximum number that can be decoded by the reader 11 (e.g., less than 16 bits). By including a serial number in the transmission, the likelihood that the reader 11 incorrectly detects two transmissions from two TAGs 15a-15c as corresponding to the same TAG 15a-15c is significantly reduced. For example, when there are five transmissions from five TAGs 15a-15c, the likelihood that at least two transmissions have pulse spaces within the same pulse space bin is about 85%. However, when the serial number is 4 bits allowing for 16 possible values, the likelihood that at least two transmissions have pulse spaces within the same pulse space bin and the same serial number is reduced to about 5%.

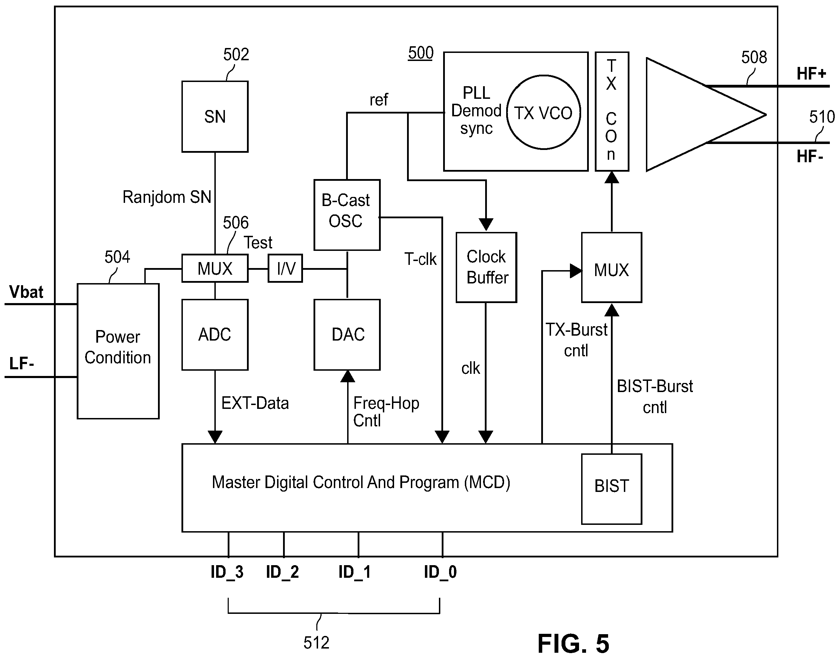

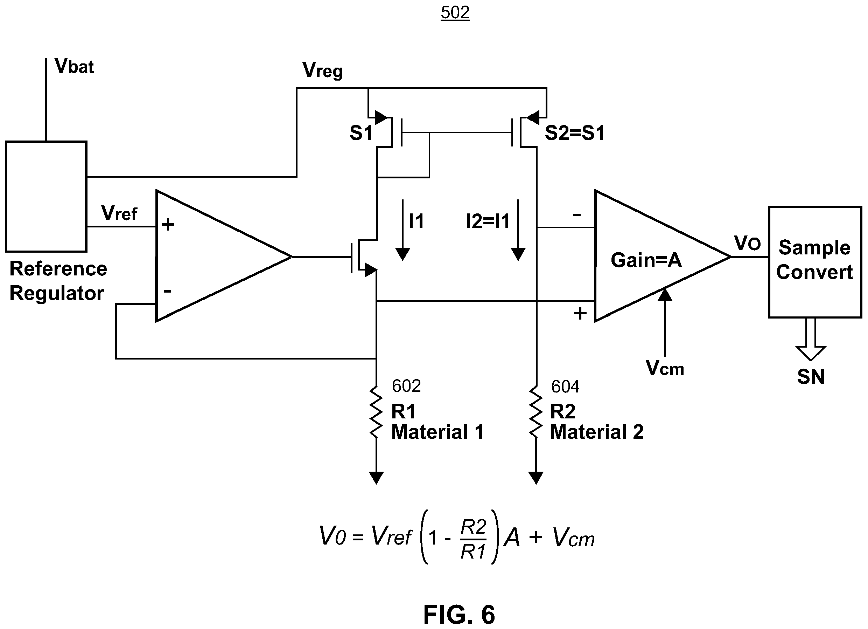

Fig. 5 illustrates an example circuit diagram 500 of an ingestible adherence sensor or TAG 15a-15c to enable the detection of multiple capsules. The circuit 500 includes a serial number generator 502 that generates a serial number to include in a serial number field of the transmission. The serial number generator 502 is described in more detail below with reference to Fig. 6. In other implementations, the serial number is obtained from external identification fields 512 (ID_0, ID_1, ID_2, ID_3). In yet other implementations, the external identification fields 512 provide an identifier of the type of medication associated with the pill 14a-14c (e.g., aspirin). The circuit also includes a power supply or battery having an input voltage ($V_{bat}$), where the battery is activated by stomach fluid. Additionally, the circuit 500 includes a power conditioning block 504 that measures the input voltage and digitizes the measured input voltage via a multiplexer (MUX) 506. The TAG 15a-15c then transmits indications of the serial number and the digitized battery level in respective fields of a transmission via out-link antenna ports 508, 510.

While the circuit diagram 500 includes a serial number generator 502, external identification fields 512, a power conditioning block 504, a MUX 506, out-link antenna ports 508, 510, and other circuity this is merely one example circuit diagram 500 for ease of illustration only. Other implementations, may include any suitable circuitry to generate or obtain a serial number, a battery level, and/or medication identifier, and transmit an encoded message that includes indications of the serial number, battery level, and/or medication identifier using a series of pulses having a particular pulse space.

Fig. 6 illustrates a detailed example circuit diagram 502 of the serial number generator as shown in Fig. 5 which generates a random serial number. By randomly generating the serial number for each TAG 15a-15c, the serial number does not need to be programmed into the TAG 15a-15c. While random numbers are typically generated using a pseudo random number generator, the pseudo random number generator will generate a new value each time the TAG 15a-15c is reset. In some scenarios, while the TAG 15a-15c is in the patient's stomach 16, the battery level may drop below a threshold voltage (e.g., when the TAG 15a-15c does not react with enough stomach fluid) and then may later increase above the threshold voltage causing the TAG 15a-15c to reset. However, when the TAG 15a-15c transmits a new value, the capsule detection system may identify the transmission as corresponding to a different TAG 15a-15c/capsule 14a-14c, resulting in a false detection.

Therefore, the serial number generator 502 is designed to initially generate a random number, but then to regenerate the same random number each time the TAG 15a-15c is reset, so that the random number does not vary over time. To accomplish this, the serial number generator 502 includes electrical components having component values which vary from part to part but do not vary over time. For example, several resistors may each have the same expected resistor value (e.g., 100 Ω). Each resistor may have a tolerance (e.g., a 5 percent tolerance), such that some of the 100 Ω resistors have actual resistances of 95 Ω, 98 Ω, 102 Ω, 105 Ω, etc. However, the actual resistances do not change over time. While some electrical component values may vary based on the temperature, the temperature inside the patient's 16 stomach reaches a steady state value of about 98 degrees Fahrenheit and does not change substantially. Therefore, when a particular resistor is selected from several resistors having the same expected resistance value, the actual resistance value for the particular resistor has some variation which may be used as an input to the generate the random number. Because the actual resistance value does not change over time, the same random number will be generated each time the TAG 15a-15c is reset.

In the example serial number generator 502, two resistors 602, 604 are selected where each resistor 602, 604 has an expected resistance value and a tolerance, such that the actual resistance values differ from the expected resistance values. Additionally, each resistor 602, 604 may be made with a different material. For example, the resistor 602 may be a polysilicon resistor, while the resistor 604 may be a diffusion resistor. The circuit 502 generates an output voltage ($V_0$) based on the actual resistance values of the resistors 602, 604. More specifically, the output voltage may be determined based on Equation 1:

$$V_0 = V_{ref}\left(1 - \frac{R2}{R1}\right)A + V_{cm}, \qquad \text{(Equation 1)}$$

where:

$V_0$ is the output voltage,
$V_{ref}$ is the reference voltage,
$V_{cm}$ is the common mode voltage,
A is the gain,
R1 is the resistance value of the fist resistor, and
R2 is the resistance value of the second resistor.

Accordingly, the average output voltage centers around $V_{cm}$, and the variation from $V_{cm}$ is based on the resistance values and the gain. By increasing the value of the gain (A), larger variations may be generated which may allow for a larger number of possible serial number values. As shown in Fig. 6, a reference regulator is also included to generate a stable value of $V_{reg}$ to eliminate variations due to battery level. The output voltage is then digitized and converted into a serial number value. For example, output voltages below a first threshold voltage may be converted into a first serial number value. Output voltages above the first threshold voltage and below a second threshold voltage may be converted into a second serial number value, etc.

While the example circuit diagram 502 as shown in Fig. 6 includes two resistors 602, 604 each having a tolerance, other implementations may include additional or alternative electrical components that have tolerances, such as capacitors, inductors, transistors, etc. Additionally, while the example circuit diagram 502 includes two electrical components 602, 604 having tolerances, other implementations may include one electrical component having a tolerance or any suitable number of electrical components having tolerances which may be used to adjust the output voltage to generate the random serial number.

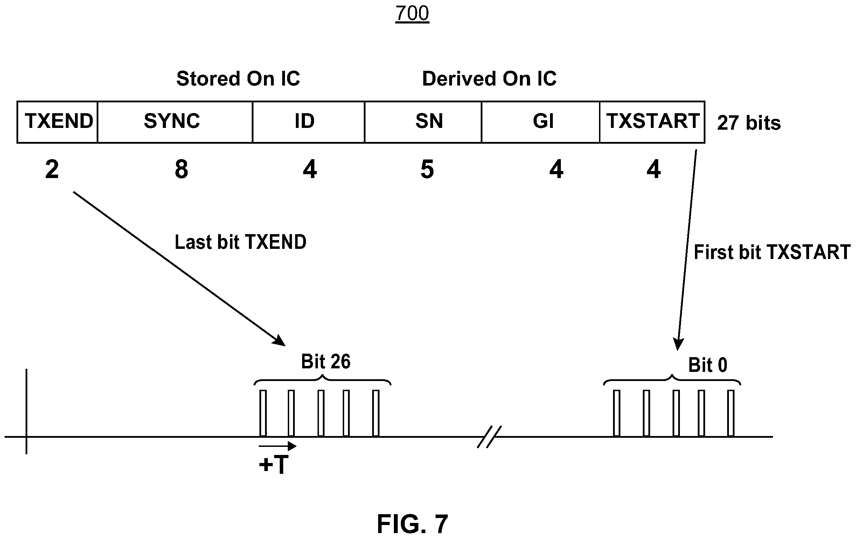

As mentioned above, when the battery in the TAG 15a-15c is activated via the patient's 16 stomach fluid, the TAG 15a-15c generates and transmits a message to the reader 11. The message may be a series of pulses encoded using pulse amplitude modulation, where for example, N pulses (e.g., five pulses) represent data one, while the lack of N consecutive pulses may represent data zero. The message may include several message fields, such as a serial number field, a battery level field, a medication identifier field, etc. Fig. 7 illustrates an example message 700 which may be transmitted by the TAG 15a-15c. The message may be a 27 bit message with a start transmission field, a battery level field (GI), a serial number field (SN), a medication identifier field (ID), a sync field, and an end transmission field. In other implementations, the message 700 may be longer or shorter than 27 bits, and may include additional or alternative message fields. Additionally, while the battery level field in the example message 700 is four bits, the serial number field is five bits, the medication identifier field is four bits, and the sync field is eight bits, this is one example message for ease of illustration only. Each of the message fields may be any suitable number of bits. For example, the serial number field may also be two or four bits.

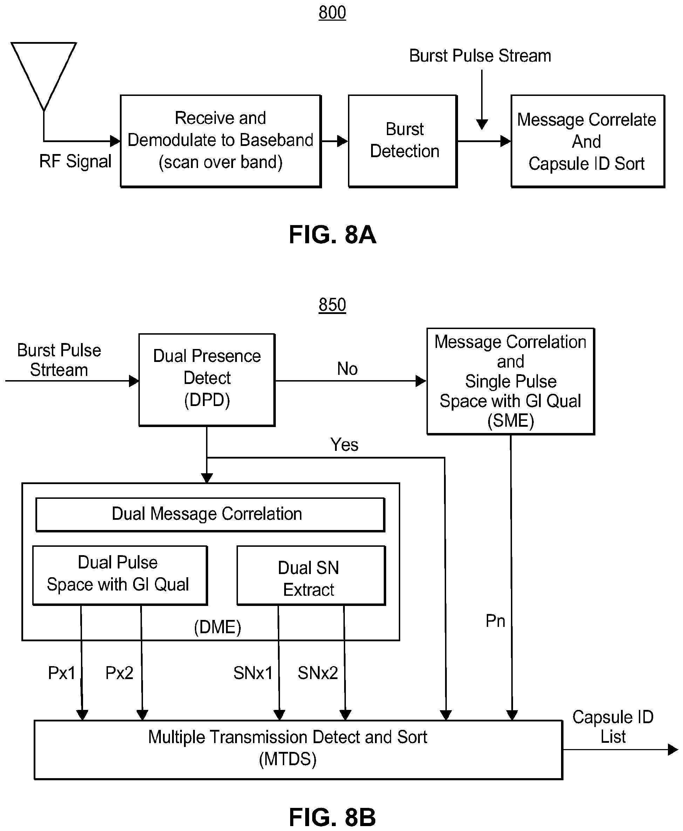

Once a message has been transmitted by a TAG 15a-15c, the reader 11 receives and decodes the message. Figs. 8A-8B illustrate example block diagrams 800, 850 of a reader 11 that receives transmissions from multiple TAGs 15a-15c. As shown in the block diagram 800 of Fig. 8A, the reader 11 receives and demodulates the message to baseband. The reader 11 may scan over a range of frequencies to determine if a message signal is present. If present, the reader 11 may pause at that frequency to receive the full transmit message. For a specific scan frequency, the pulses are detected from the baseband signal which are then provided to a message correlator. The correlator verifies the message via the known start or sync bits in the message and the full message is generated including the serial number, battery level, and ID signals.

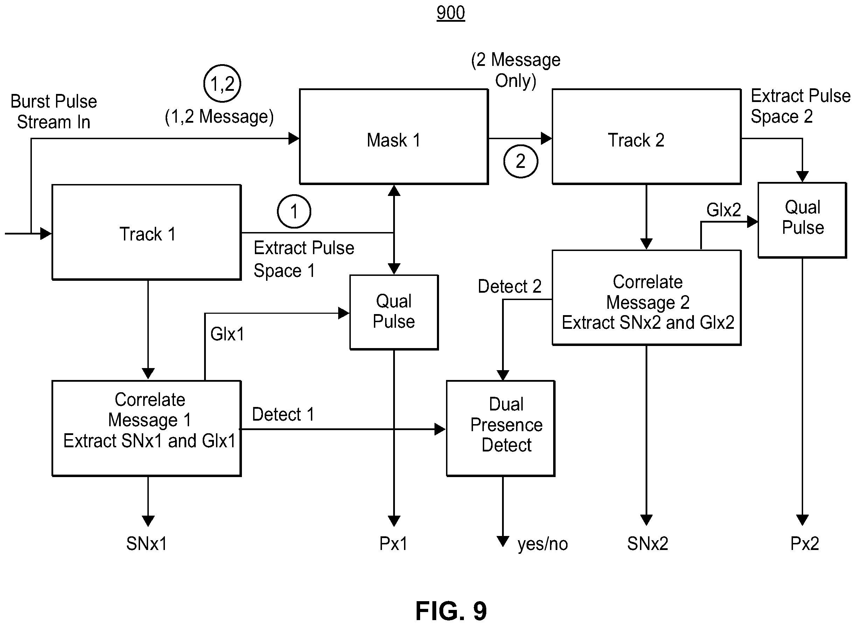

Fig. 8B illustrates a more detailed block diagram 850 of the block diagram 800 of Fig. 8A for detecting transmissions from multiple TAGs 15a-15c. In some implementations, the reader 11 includes two detectors for detecting two transmissions simultaneously or in the same detection period, which may be referred to herein as dual detection. A block diagram of an example dual detection system 900 is illustrated in Fig. 9. In the dual detection system, a first detector detects a first pulse and determines that a valid pulse sequence will not have another pulse for a threshold pulse period (e.g., 1300 $\mu$s). The first detector then ignores pulses that occur outside of a threshold time window. The threshold time window may be within a threshold guard range (e.g., 50-100 $\mu$s) of the threshold pulse period. For example, the first detector ignores pulses unless a pulse is within the threshold time window from the previous pulse (e.g., between 1200 $\mu$s and 1400 $\mu$s from the first pulse). The first detector then continues to detect subsequent pulses in this manner until the expiration of a detection period (e.g., 180 ms). The detected pulses may be identified as a first transmission or a first series of pulses.

Once a pulse is detected by the first detector, the pulse may be used to generate a blanking mask (Mask 1) which prevents the second detector from detecting the first set of pulses. The second detector then detects a second pulse which was ignored by the first detector. Like the first detector, the second detector ignores pulses that occur outside of a threshold time window, which may be within a threshold guard range (e.g., 50-100 $\mu$s) of the threshold pulse period. For example, the second detector ignores pulses unless a pulse is within the threshold time window from the previous pulse (e.g., between 1200 $\mu$s and 1400 $\mu$s from the second pulse). The second detector then continues to detect subsequent pulses in this manner until the expiration of a detection period (e.g., 180 ms). The detected pulses may be identified as a second transmission or a second series of pulses.

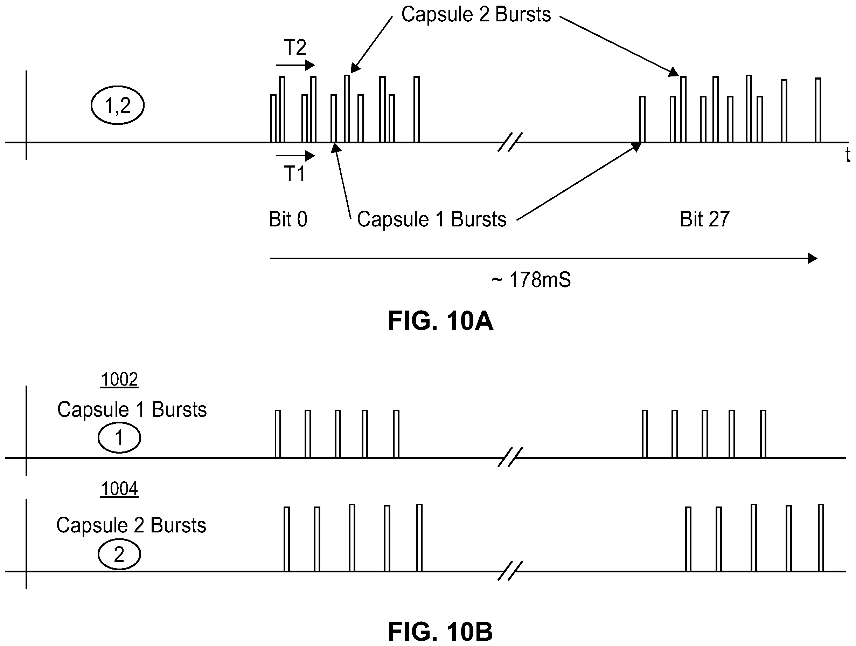

For example, as shown in Fig. 10A, the reader 11 receives pulses from two different TAGs 15a-15c. As shown in Fig. 10B, over the 178 ms detection period, the first detector detects a first series of pulses 1002 each occurring within a threshold time window from the previous pulse in the first series 1002. The second detector detects a second series of pulses 1004 each occurring within a threshold time window from the previous pulse in the second series 1004. The first and second series of pulses 1002, 1004 are identified as two different transmissions corresponding to two different TAGs 15a-15c/capsules 14a-14c.

Turning back to Fig. 8B, when both the first and second detectors detect separate transmissions, the reader 11 determines that there is a dual detection. Otherwise, if only the first detector detects a transmission, the reader 11 determines the pulse space and battery level for the transmission. When there is a dual transmission, the reader 11 decodes each of the transmissions, and identifies a first pulse space, a first battery level, and a first serial number for the first transmission, and a second pulse space, a second battery level, and a second serial number for the second transmission.

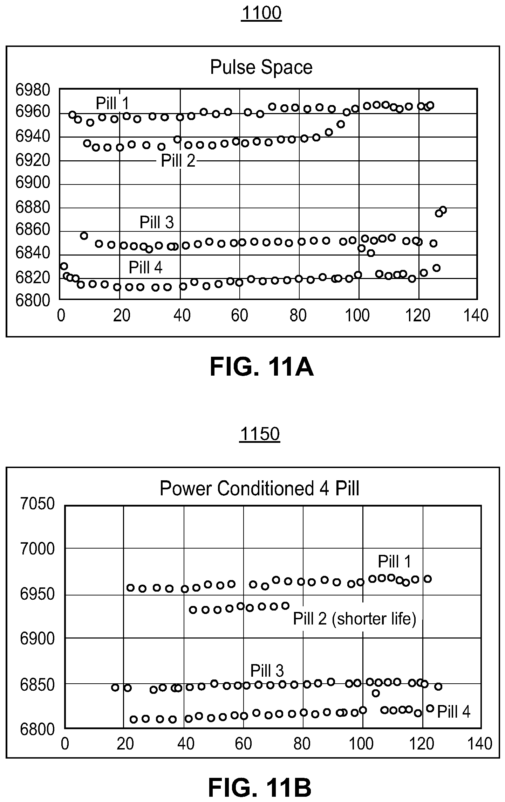

The reader 11 may adjust the pulse spaces based on the respective battery levels. For example, when a battery level is below a threshold voltage, the reader 11 may decrease the respective pulse space in accordance with the battery level. In another example, when a battery level is below a threshold voltage, the reader 11 may discard the respective pulse space. For example, Fig. 11A illustrates an example graph 1100 of pulse spaces over time for four TAGs 15a-15c corresponding to four pills 14a-14c. As shown in the graph 1100, the pulse spaces increase for Pill 2 over time, such that the pulse spaces for Pill 2 may be indistinguishable from the pulse spaces for Pill 1. This may lead to false detection. However, as shown in the example graph 1150 in Fig. 11B, by discarding pulse spaces when the battery level for Pill 2 is below a threshold voltage, the remaining pulse spaces for Pill 2 are distinguishable from the pulse spaces for Pill 1, thereby reducing the likelihood of a false detection.

While these are a few examples, the reader 11 may adjust the pulse spaces in any suitable manner based on the respective battery levels. Additionally, the reader 11 may assign each of the first and second pulse spaces to a bin having a threshold pulse space range. For example, as mentioned above, the reader 11 may convert the pulse spaces from time periods to samples based on a sample period. Then the reader 11 may assign each pulse space to a bin having a sample range (e.g., between 6772 and 6802 samples) spanning a threshold number of samples (e.g., 30 samples or about 6 $\mu$s).

After the expiration of the detection period, the first and second detectors detect third and fourth transmissions, respectively. The reader 11 then compares the characteristics of the third and fourth transmissions (e.g., pulse space, battery level, and serial number) to determine whether either or both of the third and fourth transmissions correspond to third and/or fourth TAGs 15a-15c/capsules 14a-14c. This process is then repeated over time, so that the reader 11 detects each of the TAGs 15a-15c/capsules 14a-14c.

More specifically, each time a transmission is detected having a pulse space assigned to a bin that has not been assigned previously, the transmission is identified as corresponding to an additional capsule 14a-14c and the count of the number of capsules 14a-14c is incremented. A unique pill identifier is also assigned to each transmission. For example, if five capsules 14a-14c are ingested and five transmissions are detected each having pulse spaces assigned to different bins, then the count of the number of capsules 14a-14c is five and five unique pill identifiers are assigned. If two or more pulse spaces are assigned to the same bin, then the respective serial numbers are used to determine whether the transmissions correspond to the same TAG 15a-15c/capsule 14a-14c or different TAGs 15a-15c/capsules 14a-14c.

In response to a dual detection, the reader 11 determines that there must be at least two TAGs 15a-15c and the count of the number of capsules 14a-14c is incremented to two and two unique pill identifiers are assigned to the transmissions. For subsequent dual detections, the reader 11 determines whether the pulse spaces for the subsequent dual detection are assigned to the same bin as the pulse spaces for a previous dual detection. When each of the pulse spaces is assigned to the same bin, the reader 11 determines whether a serial number in the subsequent dual detection is new. If the serial number is new, the count of the number of capsules 14a-14c is incremented and a unique pill identifier is assigned to the transmission having the new serial number. Additionally, if neither of the serial numbers in the subsequent dual detection is new, but the combination of serial numbers is new, the count of the number of capsules 14a-14c is incremented and a unique pill identifier is assigned.

For example, in a first dual detection, both transmissions have pulse spaces assigned to a first bin and serial number values of 0. Accordingly, two capsules 14a-14c are detected due to the dual detection, and the count of the number of capsules 14a-14c is incremented to two and two unique pill identifiers are assigned. In a second dual detection, both transmissions have pulse spaces assigned to the first bin and serial numbers of 0 and 1, respectively. Because 1 is a new serial number, the count of the number of capsules 14a-14c is incremented to three and a unique pill identifier is assigned. In a third dual detection, both transmissions have pulse spaces assigned to the first bin and serial numbers of 1 and 0, respectively. Because neither of the serial numbers is new and the combination of serial numbers is not new, the count is not incremented. In a fourth dual detection, both transmissions have pulse spaces assigned to the first bin and serial numbers of 0 and 1, respectively. Because neither of the serial numbers is new and the combination of serial numbers is not new, the count is not incremented. In a fifth dual detection, both transmissions have pulse spaces assigned to the first bin and serial numbers of 0 and 0, respectively. Because neither of the serial numbers is new and the combination of serial numbers is not new, the count is not incremented. As a result, the final count is three capsules 14a-14c.

In another example, in a first dual detection, both transmissions have pulse spaces assigned to a first bin and serial number values of 0 and 1, respectively. Accordingly, two capsules 14a-14c are detected due to the dual detection and/or the different serial numbers, and the count of the number of capsules 14a-14c is incremented to two and two unique pill identifiers are assigned. In a second dual detection, both transmissions have pulse spaces assigned to the first bin and serial numbers of 1 and 0, respectively. Because neither of the serial numbers is new and the combination of serial numbers is not new, the count is not incremented. In a third dual detection, both transmissions have pulse spaces assigned to the first bin and serial numbers of 0 and 1, respectively. Because neither of the serial numbers is new and the combination of serial numbers is not new, the count is not incremented. In a fourth dual detection, both transmissions have pulse spaces assigned to the first bin and serial numbers of 0 and 0, respectively. While 0 is not a new serial number, the combination of 0 and 0 is new. Therefore, the count of the number of capsules 14a-14c is incremented to three and a unique pill identifier is assigned. In a fifth dual detection, both transmissions have pulse spaces assigned to the first bin and serial numbers of 0 and 1, respectively. Because neither of the serial numbers is new and the combination of serial numbers is not new, the count is not incremented. As a result, the final count is three capsules 14a-14c.

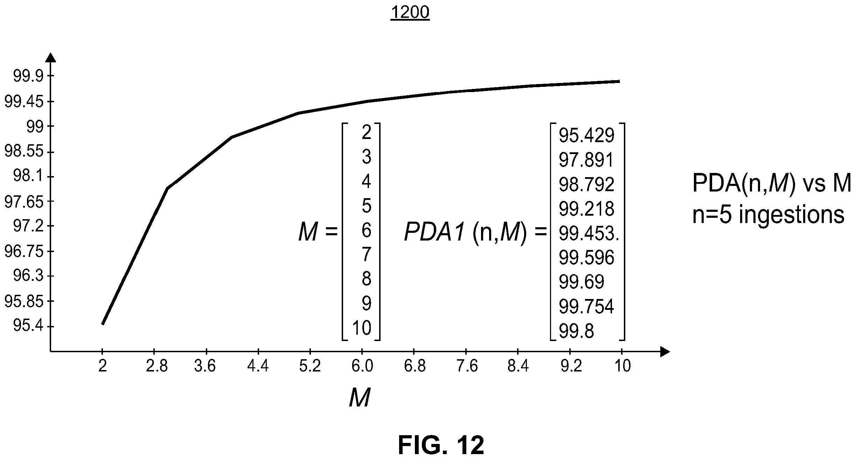

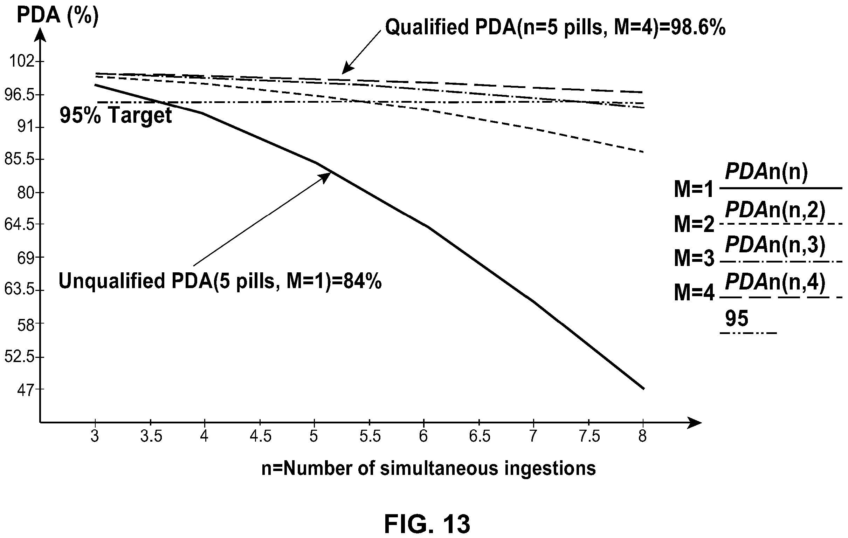

Figs. 12 and 13 depict performance metrics of the capsule detection system 10 in identifying multiple capsules 14a-14c. More specifically, Fig. 12 illustrates an example graph 1200 depicting the accuracy of the multiple capsule detection system 10 detecting five capsules 14a-14c as a function of the number of possible serial number values (M) when using dual detection. When using one bit for the serial number or two possible serial number values, the multiple capsule detection system 10 detects five capsules with about 95.4% accuracy. When using two bits for the serial number or four possible serial number values, the multiple capsule detection system 10 detects five capsules with about 98.8% accuracy. When using three bits for the serial number or eight possible serial number values, the multiple capsule detection system 10 detects five capsules with about 99.7% accuracy. Dual detection significantly improves the performs of the system 10. For example, without dual detection and when using two bits for the serial number or four possible serial number values, the multiple capsule detection system 10 detects five capsules with only about 79% accuracy.

Fig. 13 illustrates an example graph 1300 depicting the accuracy of the multiple capsule detection system 10 as a function of the number of capsules (n) and the number of possible serial number values (M) when using dual detection. If no serial number is used (M =1), the accuracy decreases significantly as the number of capsules increases. The multiple capsule detection system 10 detects five capsules with only about 84% accuracy. When using two bits for the serial number or four possible serial number values (M=4), up to eight capsules may be detected with over 95% accuracy.

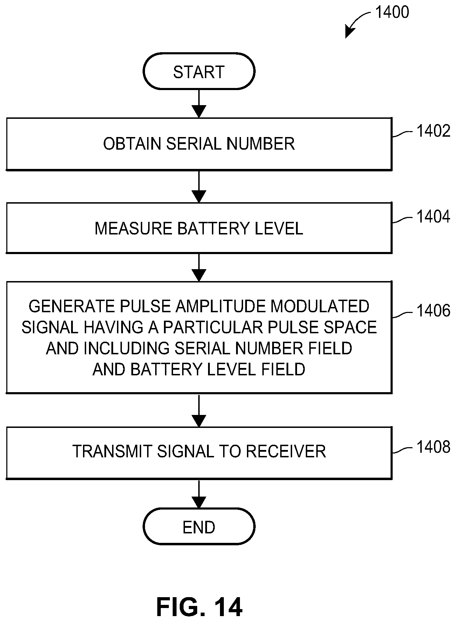

Fig. 14 depicts a flow diagram of an example method 1400 for enabling detection of a plurality of capsule ingestions. The method 1400 may be implemented by a TAG 15a-15c.

At block 1402, a serial number is obtained. The serial number may be programmed into the TAG 15a-15c or may be randomly generated. In some implementations, the serial number is randomly generated using a serial number generator 502 circuit as shown in Fig. 6. The serial number may be one, two, three, four, or five bits. In other implementations, the serial number may be a number larger than five bits, but less than a maximum number that can be decoded by a reader 11 (e.g., less than 16 bits).

Then at block 1404, the battery level in the TAG 15a-15c is measured. For example, the TAG 15a-15c may measure the input voltage from the battery and may digitize the measured input voltage, for example with a MUX. The TAG 15a-15c may then generate a transmission that includes the battery level and the serial number (block 1406). For example, the transmission may include a message having a battery level field and a serial number field. The transmission may be a series of pulses encoded using pulse amplitude modulation, where for example, N pulses (e.g., five pulses) represent data one, while the lack of N consecutive pulses may represent data zero. Each of the pulses in the series of pulse modulated RF signals may have a duration of about 1 to 4 $\mu$s and a pulse period or pulse space of between 1 and 1.5 ms. The pulses may be generated with a particular pulse space (e.g., 1.32 ms) to identify the transmission as corresponding to an additional capsule 14a-14c.

Then the TAG 15a-15c may transmit the transmission to a reader 11 (block 1408). The reader 11 may then decode the transmission and identify whether the transmission corresponds to an additional capsule 14a-14c based on the pulse space, the battery level, and/or the serial number.

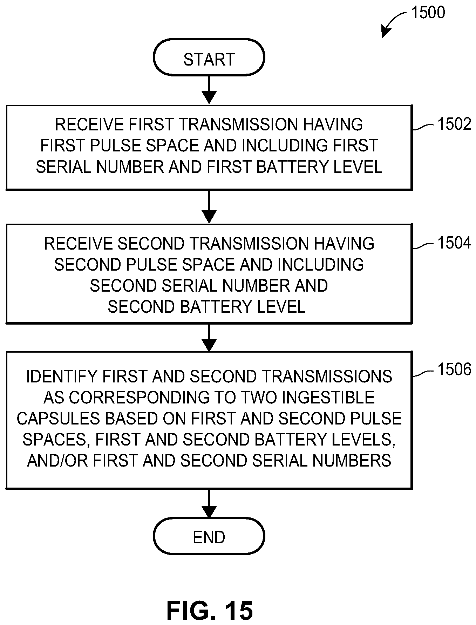

Fig. 15 depicts a flow diagram of an example method 1500 for identifying a plurality of capsule ingestions. The method 1500 may be implemented by a reader 11.

At block 1502, a first transmission is received having a first pulse space and including a first serial number and a first battery level. More specifically, the reader 11 receives and demodulates the first transmission to baseband. The reader 11 may scan over a range of frequencies to determine if a transmission is present. If present, the reader 11 may pause at that frequency to receive the full transmit message. For a specific scan frequency, the pulses are detected from the baseband signal which are then provided to a message correlator. The correlator verifies the message via the known start or sync bits in the message and the full message is generated including the serial number, battery level, and ID signals.

A second transmission is also received having a second pulse space and including a second serial number and a second battery level (block 1504). In some implementations, the first and second transmissions are detected simultaneously or in the same detection period by first and second detectors using dual detection. In the dual detection system, the first detector detects a first pulse and determines that a valid pulse sequence will not have another pulse for a threshold pulse period (e.g., 1300 $\mu$s). The first detector then ignores pulses that occur outside of a threshold time window, which may be within a threshold guard range (e.g., 50-100 $\mu$s) of the threshold pulse period. The first detector then continues to detect subsequent pulses in this manner until the expiration of a detection period (e.g., 180 ms).

Once a pulse is detected by the first detector, the pulse may be used to generate a blanking mask (Mask 1) which prevents the second detector from detecting the first set of pulses. The second detector then detects a second pulse which was ignored by the first detector. Like the first detector, the second detector ignores pulses that occur outside of a threshold time window, which may be within a threshold guard range (e.g., 50-100 $\mu$s) of the threshold pulse period. The second detector then continues to detect subsequent pulses in this manner until the expiration of a detection period (e.g., 180 ms).

At block 1506, the reader 11 determines that the two transmissions correspond to two capsules 14a-14c based on the first and second pulse spaces, the first and second battery levels, and/or the first and second serial numbers. For example, in the dual detection system where the two transmissions were detected simultaneously or in the same detection period, the reader 11 may determine that the two transmissions correspond to two capsules 14a-14c. In another example, the reader 11 may assign the first and second pulse spaces to bins having threshold pulse space ranges. If the first and second pulse spaces are assigned to different bins, the reader 11 may determine that the two transmissions correspond to two capsules 14a-14c. If the first and second pulse spaces are assigned to the same bin, the reader 11 may compare the first and second serial numbers. If the first and second serial numbers are different, the reader 11 may determine that the two transmissions correspond to two capsules 14a-14c. Additionally, the reader 11 may discard a pulse space when the battery level is below a threshold voltage. On the other hand, the reader 11 may determine that the two transmissions correspond to two capsules 14a-14c when the first and second pulse spaces are assigned to different bins and the first and second battery levels are at or above the threshold voltage.

In some cases, a computing device may be used to implement various modules, circuits, systems, methods, or algorithm steps disclosed herein. As an example, all or part of a module, circuit, system, method, or algorithm disclosed herein may be implemented or performed by a general-purpose single- or multi-chip processor, a digital signal processor (DSP), an ASIC, a FPGA, any other suitable programmable-logic device, discrete gate or transistor logic, discrete hardware components, or any suitable combination thereof. A general-purpose processor may be a microprocessor, or, any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

In particular embodiments, one or more implementations of the subject matter described herein may be implemented as one or more computer programs (e.g., one or more modules of computer-program instructions encoded or stored on a computer-readable non-transitory storage medium). As an example, the steps of a method or algorithm disclosed herein may be implemented in a processor-executable software module which may reside on a computer-readable non-transitory storage medium. In particular embodiments, a computer-readable non-transitory storage medium may include any suitable storage medium that may be used to store or transfer computer software and that may be accessed by a computer system. Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs (e.g., compact discs (CDs), CD-ROM, digital versatile discs (DVDs), blue-ray discs, or laser discs), optical disc drives (ODDs), magneto-optical discs, magneto-optical drives, floppy diskettes, floppy disk drives (FDDs), magnetic tapes, flash memories, solid-state drives (SSDs), RAM, RAM-drives, ROM, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

In some cases, certain features described herein in the context of separate implementations may also be combined and implemented in a single implementation. Conversely, various features that are described in the context of a single implementation may also be implemented in multiple implementations separately or in any suitable sub-combination. Moreover, although features may be described above as acting in certain combinations and even initially claimed as such, one or more features from a claimed combination may in some cases be excised from the combination, and the claimed combination may be directed to a sub-combination or variation of a sub-combination.

While operations may be depicted in the drawings as occurring in a particular order, this should not be understood as requiring that such operations be performed in the particular order shown or in sequential order, or that all operations be performed. Further, the drawings may schematically depict one more example processes or methods in the form of a flow diagram or a sequence diagram. However, other operations that are not depicted may be incorporated in the example processes or methods that are schematically illustrated. For example, one or more additional operations may be performed before, after, simultaneously with, or between any of the illustrated operations. Moreover, one or more operations depicted in a diagram may be repeated, where appropriate. Additionally, operations depicted in a diagram may be performed in any suitable order. Furthermore, although particular components, devices, or systems are described herein as carrying out particular operations, any suitable combination of any suitable components, devices, or systems may be used to carry out any suitable operation or combination of operations. In certain circumstances, multitasking or parallel processing operations may be performed. Moreover, the separation of various system components in the implementations described herein should not be understood as requiring such separation in all implementations, and it should be understood that the described program components and systems may be integrated together in a single software product or packaged into multiple software products.

Various implementations have been described in connection with the accompanying drawings. However, it should be understood that the figures may not necessarily be drawn to scale. As an example, distances or angles depicted in the figures are illustrative and may not necessarily bear an exact relationship to actual dimensions or layout of the devices illustrated.

The scope of this disclosure encompasses all changes, substitutions, variations, alterations, and modifications to the example embodiments described or illustrated herein that a person having ordinary skill in the art would comprehend. The scope of this disclosure is not limited to the example embodiments described or illustrated herein. Moreover, although this disclosure describes or illustrates respective embodiments herein as including particular components, elements, functions, operations, or steps, any of these embodiments may include any combination or permutation of any of the components, elements, functions, operations, or steps described or illustrated anywhere herein that a person having ordinary skill in the art would comprehend.

The term "or" as used herein is to be interpreted as an inclusive or meaning any one or any combination, unless expressly indicated otherwise or indicated otherwise by context. Therefore, herein, the expression "A or B" means "A, B, or both A and B." As another example, herein, "A, B or C" means at least one of the following: A; B; C; A and B; A and C; B and C; A, B and C. An exception to this definition will occur if a combination of elements, devices, steps, or operations is in some way inherently mutually exclusive.

As used herein, words of approximation such as, without limitation, "approximately, "substantially," or "about" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as having the required characteristics or capabilities of the unmodified feature. In general, but subject to the preceding discussion, a numerical value herein that is modified by a word of approximation such as "approximately" may vary from the stated value by $\pm 0.5\%$, $\pm 1\%$, $\pm 2\%$, $\pm 3\%$, $\pm 4\%$, $\pm 5\%$, $\pm 10\%$, $\pm 12\%$, or $\pm 15\%$.

As used herein, the terms "first," "second," "third," etc. may be used as labels for nouns that they precede, and these terms may not necessarily imply a particular ordering (e.g., a particular spatial, temporal, or logical ordering). As an example, a system may be described as determining a "first result" and a "second result," and the terms "first" and "second" may not necessarily imply that the first result is determined before the second result.

As used herein, the terms "based on" and "based at least in part on" may be used to describe or present one or more factors that affect a determination, and these terms may not exclude additional factors that may affect a determination. A determination may be based solely on those factors which are presented or may be based at least in part on those factors. The phrase "determine A based on B" indicates that B is a factor that affects the determination of A.

In some instances, other factors may also contribute to the determination of A. In other instances, A may be determined based solely on B.

**Claims**

1.  A method for enabling detection of a plurality of capsule ingestions, the method comprising:

    for each of a plurality of ingestible capsules (14a-14c) including a transmitter and processing hardware to generate and transmit signals:

    obtaining, by the processing hardware, a serial number for distinguishing between the plurality of ingestible capsules (14a-14c);
    generating, by the processing hardware, a signal indicating that the capsule (14a-14c) has been ingested, the signal including a series of pulses having a particular pulse space and indicating the serial number; and
    transmitting the signal to a receiver via the transmitter,

    wherein each of the plurality of ingestible capsules (14a-14c) is identified based on the particular pulse space and the serial number for the ingestible capsule (14a-14c).

2.  The method of claim 1, further comprising:

    measuring, by the processing hardware, a battery level of a battery providing power to the processing hardware, wherein generating the signal further includes generating an indication of the battery level, and
    wherein each of the plurality of ingestible capsules (14a-14c) is further identified based on the battery level, and, wherein the battery is powered by chemical energy from fluid within a patient's gastrointestinal tract.

3.  The method of any one of claims 1-2, wherein obtaining the serial number includes generating, by the processing hardware, a random number as the serial number.

4.  The method of any one of claims 1-3, wherein the signal is encoded using pulse amplitude modulation, and, wherein generating the signal includes generating, by the processing hardware, a message encoded by the pulse amplitude modulated signal including a serial number field and a battery level field.

5.  A method for identifying a plurality of capsule ingestions, the method comprising:

    receiving, by processing hardware in a receiver from a first ingestible capsule (14a-14c), a first transmission having a first series of pulses with a first pulse space and including an indication of a first serial number;
    receiving, by the processing hardware from a second ingestible capsule (14a-14c), a second transmission having a second series of pulses with a second pulse space and including an indication of a second serial number;
    identifying, by the processing hardware, the first and second transmissions as corresponding to at least two ingestible capsules (14a-14c) based on (i) the first and second pulse spaces, and (ii) the first and second serial numbers.

6.  The method of claim 5, further comprising:

    obtaining, by the processing hardware, a plurality of bins each corresponding to a different range of pulse space values;
    assigning, by the processing hardware, the first pulse space to a first one of the plurality of bins;
    assigning, by the processing hardware, the second pulse space to a second one of the plurality of bins, wherein the first and second transmissions are identified as corresponding to at least two ingestible capsules (14a-14c) based on the first and second bins, and, wherein the first and second transmissions are identified as corresponding to at least two ingestible capsules (14a-14c) in response to determining that the first and second bins are different.

7.  The method of claim 6, wherein the first and second transmissions are received in a same detection period, and further comprising:

detecting, by a first detector in the receiver, the first transmission by identifying a first series of pulses spaced apart by at least a threshold pulse space;

masking, by a second detector in the receiver, the first transmission; and

after masking the first transmission, detecting, by the second detector, the second transmission as a separate transmission by identifying a second series of pulses spaced apart by the at least threshold pulse space.

8. The method of claim 7, wherein the first and second transmissions are identified as corresponding to at least two ingestible capsules (14a-14c) further based on detecting the first and second transmissions as separate transmissions.

9. The method of claim 7, wherein the first and second transmissions are detected within a first detection period, and further comprising in a second detection period:

detecting, by the first detector, a third transmission by identifying a third series of pulses spaced apart by at least a threshold pulse space;

masking, by the second detector, the third transmission;

after masking the third transmission, detecting, by the second detector, a fourth transmission as a separate transmission from the third transmission by identifying a fourth series of pulses spaced apart by the at least threshold pulse space;

assigning, by the processing hardware, a third pulse space for the third transmission to a third one of the plurality of bins; and

assigning, by the processing hardware, a fourth pulse space for the fourth transmission to a fourth one of the plurality of bins.

10. The method of claim 9, wherein the first, second, third, and fourth bins are the same bin, and further comprising: determining, by the processing hardware, whether the third or fourth transmissions correspond to a third ingestible capsule (14a-14c) in response to determining at least one of:

that a third serial number for the third transmission or a fourth serial number for the fourth transmission is different from the first and second serial numbers, or

that a combination of the third and fourth serial numbers is different from a combination of the first and second serial numbers.

11. The method of claim 5, wherein the first and second transmissions are identified as corresponding to at least two ingestible capsules (14a-14c) in response to determining that the first and second serial numbers are different.

12. The method of claim 5, wherein:

receiving the first transmission further includes receiving, by the processing hardware, an indication of a first battery level of the first ingestible capsule (14a-14c), and

receiving the second transmission further includes receiving, by the processing hardware, an indication of a second battery level of the second ingestible capsule (14a-14c), , wherein the first and second transmissions are identified as corresponding to at least two ingestible capsules (14a-14c) further based on the first and second battery levels.

13. An ingestible bio-telemetry tag system comprising:

a capsule (14a-14c);

a transmitter; and

processing hardware coupled to the capsule (14a-14c) and the transmitter including:

an electrical component having an expected component value and a tolerance, wherein an actual component value for the electrical component does not vary over time; and

the processing hardware configured to:

generate an output voltage in accordance with the actual component value for the electrical component; and

generate a random serial number based on the output voltage, wherein the random serial number remains the same each time the ingestible bio-telemetry tag system is reset.

**14.** The ingestible bio-telemetry tag system of claim 13, wherein the electrical component is a first electrical component made from a first material, and the processing hardware further includes:
a second electrical component made from a second material different from the first material.

**15.** The ingestible bio-telemetry tag system of claim 13, wherein the electrical component is a resistor.

## Patentansprüche

**1.** Verfahren zur Ermöglichung des Nachweises einer Vielzahl von Kapselaufnahmen, wobei das Verfahren umfasst:

für jede einer Vielzahl von einnehmbaren Kapseln (14a-14c) mit einem Sender und einer Verarbeitungshardware zur Erzeugung und Übertragung von Signalen:

Erhalten einer Seriennummer durch die Verarbeitungshardware zur Unterscheidung zwischen der Vielzahl der einnehmbaren Kapseln (14a-14c);
Erzeugen eines Signals durch die Verarbeitungshardware, das anzeigt, dass die Kapsel (14a-14c) eingenommen wurde, wobei das Signal eine Reihe von Impulsen mit einem bestimmten Impulsabstand enthält und die Seriennummer angibt; und
Übertragen des Signals über den Sender an einen Empfänger,

wobei jede der Vielzahl von einnehmbaren Kapseln (14a-14c) auf der Grundlage von dem speziellen Impulsraum und der Seriennummer für die einnehmbare Kapsel (14a-14c) identifiziert wird.

**2.** Verfahren nach Anspruch 1, ferner umfassend:

Messen des Batteriestands einer Batterie, die die Verarbeitungshardware mit Strom versorgt, durch die Verarbeitungshardware,
wobei das Erzeugen des Signals ferner das Erzeugen einer Anzeige des Batteriestands umfasst, und
wobei jede der Vielzahl von einnehmbaren Kapseln (14a-14c) ferner auf der Grundlage des Batteriestandes identifiziert wird, und wobei die Batterie durch chemische Energie aus Flüssigkeit im Magen-Darm-Trakt eines Patienten betrieben wird.

**3.** Das Verfahren nach einem der Ansprüche 1-2, wobei das Ermitteln der Seriennummer das Erzeugen einer Zufallszahl als Seriennummer durch die Verarbeitungshardware umfasst.

**4.** Verfahren nach einem der Ansprüche 1-3, wobei das Signal unter Verwendung von Puls-Amplituden-Modulation kodiert wird, und wobei das Erzeugen des Signals das Erzeugen einer durch das Puls-Amplituden-modulierte Signal kodierten Nachricht einschließlich eines Seriennummernfeldes und eines Batteriestandsfeldes durch die Verarbeitungshardware umfasst.

**5.** Verfahren zum Identifizieren einer Vielzahl von Kapselaufnahmen, wobei das Verfahren umfasst:

Empfangen, durch Verarbeitungshardware in einem Empfänger, einer ersten Übertragung, die eine erste Reihe von Impulsen mit einem ersten Impulsabstand aufweist und eine Angabe einer ersten Seriennummer enthält von einer ersten einnehmbaren Kapsel (14a-14c);
Empfangen, durch die Verarbeitungshardware, einer zweiten Übertragung von einer zweiten einnehmbaren Kapsel (14a-14c), die eine zweite Reihe von Impulsen mit einem zweiten Impulsabstand aufweist und eine Angabe einer zweiten Seriennummer enthält;
Identifizieren der ersten und zweiten Übertragungen durch die Verarbeitungshardware als zu mindestens zwei einnehmbaren Kapseln (14a-14c) gehörend, basierend auf (i) dem ersten und zweiten Impulsabstand und (ii) der ersten und zweiten Seriennummer.

**6.** Verfahren nach Anspruch 5, ferner umfassend:

Erhalten, durch die Verarbeitungshardware, eine Vielzahl von Bins, die jeweils einem anderen Bereich von Impulsraumwerten entsprechen;
Zuordnen, durch die Verarbeitungshardware, des ersten Impulsraums zu einem ersten der mehreren Bins;

Zuordnen, durch die Verarbeitungshardware, des zweiten Impulsraums zu einem zweiten der mehreren Bins, wobei die ersten und zweiten Übertragungen als mindestens zwei einnehmbaren Kapseln (14a-14c) entsprechend identifiziert werden, basierend auf den ersten und zweiten Bins, und wobei die ersten und zweiten Übertragungen als mindestens zwei einnehmbaren Kapseln (14a-14c) entsprechend identifiziert werden, in Reaktion auf die Feststellung, dass die ersten und zweiten Bins unterschiedlich sind.

7. Verfahren nach Anspruch 6, wobei die erste und die zweite Übertragung im selben Erfassungszeitraum empfangen werden, und ferner umfassend:

Erkennen der ersten Übertragung durch einen ersten Detektor im Empfänger, indem eine erste Reihe von Impulsen identifiziert wird, die mindestens um einen Schwellenimpulsabstand voneinander getrennt sind; Maskieren der ersten Übertragung durch einen zweiten Detektor im Empfänger; und nach Maskieren der ersten Übertragung, Erkennen der zweiten Übertragung durch den zweiten Detektor als separate Übertragung durch Identifizierem einer zweiten Reihe von Impulsen, die durch den Mindestschwellen-Impulsabstand voneinander getrennt sind.

8. Verfahren nach Anspruch 7, wobei Identifizieren der ersten und zweiten Übertragungen als mindestens zwei einnehmbaren Kapseln (14a-14c) entsprechend ferner auf dem Erkennen der ersten und zweiten Übertragungen als separate Übertragungen basiert.

9. Verfahren nach Anspruch 7, wobei die ersten und zweiten Übertragungen innerhalb eines ersten Erfassungszeitraums erfasst werden, und ferner in einem zweiten Erfassungszeitraum umfasst:

Erkennen einer dritten Übertragung durch den ersten Detektor, indem eine dritte Reihe von Impulsen identifiziert wird, die mindestens um einen Schwellenimpulsabstand voneinander entfernt sind; Maskieren der dritten Übertragung durch den zweiten Detektor; nach Maskieren der dritten Übertragung, Erkennen einer vierten Übertragung durch den zweiten Detektor als eine von der dritten Übertragung getrennte Übertragung durch Identifizierung einer vierten Reihe von Impulsen, die durch den Mindestschwellen-Impulsabstand voneinander getrennt sind; Zuweisen eines dritten Impulsraums für die dritte Übertragung zu einem dritten der Vielzahl von Bins durch die Verarbeitungshardware; und Zuordnen eines vierten Impulsraums für die vierte Übertragung zu einem vierten der mehreren Bins durch die Verarbeitungshardware.

10. Verfahren nach Anspruch 9, wobei der erste, zweite, dritte und vierte Bin derselbe Bin sind, und ferner umfassend: Bestimmen, durch die Verarbeitungshardware, ob die dritte oder vierte Übertragung einer dritten einnehmbaren Kapsel (14a-14c) entspricht, als Reaktion auf die Bestimmung von mindestens einem der folgenden Punkte:

dass sich eine dritte Seriennummer für die dritte Übertragung oder eine vierte Seriennummer für die vierte Übertragung von der ersten und zweiten Seriennummer unterscheidet, oder dass sich eine Kombination aus der dritten und vierten Seriennummer von einer Kombination aus der ersten und zweiten Seriennummer unterscheidet.

11. Verfahren nach Anspruch 5, wobei die erste und die zweite Übertragung als mindestens zwei einnehmbaren Kapseln (14a-14c) entsprechend identifiziert werden, wenn festgestellt wird, dass die erste und die zweite Seriennummer unterschiedlich sind.

12. Verfahren nach Anspruch 5, wobei:

das Empfangen der ersten Übertragung ferner das Empfangen einer Anzeige eines ersten Batteriestands der ersten einnehmbaren Kapsel (14a-14c) durch die Verarbeitungshardware umfasst, und das Empfangen der zweiten Übertragung ferner das Empfangen einer Anzeige eines zweiten Batteriestands der zweiten einnehmbaren Kapsel (14a-14c) durch die Verarbeitungshardware einschließt, wobei die erste und die zweite Übertragung als mindestens zwei einnehmbaren Kapseln (14a-14c) entsprechend identifiziert werden, ferner basierend auf dem ersten und dem zweiten Batteriestand.

13. Ein verschluckbares Bio-Telemetrie-Etiketten-System, das Folgendes umfasst:

eine Kapsel (14a-14c);

einen Sender; und

Verarbeitungshardware, die mit der Kapsel (14a-14c) und dem Sender verbunden ist, einschließlich:

ein elektrisches Bauteil mit einem erwarteten Bauteilwert und einer Toleranz, wobei sich ein tatsächlicher Bauteilwert für das elektrische Bauteil im Laufe der Zeit nicht ändert; und

die Verarbeitungshardware ist eingerichtet zum:

Erzeugen einer Ausgangsspannung in Übereinstimmung mit dem tatsächlichen Komponentenwert für die elektrische Komponente; und

Erzeugen einer zufälligen Seriennummer auf der Grundlage der Ausgangsspannung, wobei die zufällige Seriennummer jedes Mal gleich bleibt, wenn das einnehmbare Bio-Telemetrie-Etiketten-System zurückgesetzt wird.

14. Das verschluckbare Bio-Telemetrie-Etiketten-System nach Anspruch 13, wobei das elektrische Bauteil ein erstes elektrisches Bauteil ist, das aus einem ersten Material hergestellt ist, und die Verarbeitungshardware ferner umfasst: ein zweites elektrisches Bauteil aus einem zweiten Material, das sich von dem ersten Material unterscheidet.

15. Das verschluckbare Bio-Telemetrie-Etiketten-System nach Anspruch 13, wobei das elektrische Bauteil ein Widerstand ist.


**Revendications**

1. Un procédé permettant la détection d'une pluralité d'ingestions de capsules, le procédé comprenant :

pour chacune d'une pluralité de capsules ingestibles (14a-14c) comprenant un émetteur et un hardware de traitement pour générer et émettre des signaux :

l'obtention, par le hardware de traitement, d'un numéro de série permettant de distinguer entre la pluralité de capsules ingestibles (14a-14c) ;

la génération, par le hardware de traitement, d'un signal indiquant que la capsule (14a-14c) a été ingérée, le signal comprenant une série d'impulsions avec un intervalle particulier entre impulsions et indiquant le numéro de série ; et

l'émission du signal vers un récepteur via l'émetteur,

dans lequel chacune de la pluralité de capsules ingestibles (14a-14c) est identifiée sur la base de l'intervalle particulier entre impulsions et du numéro de série pour la capsule ingestible (14a-14c).

2. Le procédé de la revendication 1, comprenant en outre :

la mesure, par le hardware de traitement, d'un niveau de batterie d'une batterie délivrant une alimentation au hardware de traitement,

dans lequel la génération du signal comprend en outre la génération d'une indication du niveau de batterie, et dans lequel chacune de la pluralité de capsules ingestibles (14a-14c) est en outre identifiée sur la base du niveau de batterie, et dans lequel la batterie est alimentée par une énergie chimique provenant d'un fluide au sein d'un tractus gastrointestinal du patient.

3. Le procédé de l'une des revendications 1 à 2, dans lequel l'obtention du numéro de série comprend la génération, par le hardware de traitement, d'un numéro aléatoire en tant que numéro de série.

4. Le procédé de l'une des revendications 1 à 3, dans lequel le signal est codé à l'aide d'une modulation d'amplitude d'impulsion, et dans lequel la génération du signal comprend la génération, par le hardware de traitement, d'un message codé par le signal modulé en amplitude d'impulsion, incluant un champ de numéro de série et un champ de niveau de batterie.

5. Un procédé d'identification d'une pluralité d'ingestions de capsules, le procédé comprenant :

la réception, par un hardware de traitement dans un récepteur, en provenance d'une première capsule ingestible (14a-14c), d'une première transmission avec une première série d'impulsions ayant un premier intervalle entre impulsions et comprenant une indication d'un premier numéro de série ;

la réception, par le hardware de traitement, en provenance d'une seconde capsule ingestible (14a-14c), d'une seconde transmission avec une seconde série d'impulsions ayant un second intervalle entre impulsions et comprenant une indication d'un second numéro de série ;

l'identification, par le hardware de traitement, de la première et de la seconde transmission comme correspondant à au moins deux capsules ingestibles (14a-14c), sur la base (i) du premier et du second intervalle entre impulsions, et (ii) du premier et du second numéro de série.

6. Le procédé de la revendication 5, comprenant en outre :

l'obtention, par le hardware de traitement, d'une pluralité de bins correspondant chacun à une plage différente de valeurs d'intervalle entre impulsions ;

l'attribution, par le hardware de traitement, du premier intervalle entre impulsions à un premier bin de la pluralité de bins ;

l'attribution, par le hardware de traitement, du second intervalle entre impulsions à un second bin de la pluralité de bins,

dans lequel la première et la seconde transmission sont identifiées comme correspondant à au moins deux capsules ingestibles (14a-14c) sur la base du premier et du second bin, et dans lequel la première et la seconde transmission sont identifiées comme correspondant à au moins deux capsules ingestibles (14a-14c) en réponse à la détermination que le premier et le second bin sont différents.

7. Le procédé de la revendication 6, dans lequel la première et la seconde transmission sont reçues dans une même période de détection, et comprenant en outre :

la détection, par un premier détecteur dans le récepteur, de la première transmission par identification d'une première série d'impulsions espacées entre elles d'au moins un intervalle seuil entre impulsions ;

le masquage, par un second détecteur dans le récepteur, de la première transmission ; et

après masquage de la première transmission, la détection, par le second détecteur, de la seconde transmission comme étant une transmission distincte, par identification d'une seconde série d'impulsions espacées entre elles de l'au moins un intervalle seuil entre impulsions.

8. Le procédé de la revendication 7, dans lequel la première et la seconde transmission sont identifiées comme correspondant à au moins deux capsules ingestibles (14a-14c) sur la base en outre de la détection de la première et de la seconde transmission comme étant des transmissions distinctes.

9. Le procédé de la revendication 7, dans lequel la première et la seconde transmission sont détectées à l'intérieur d'une première période de détection, et comprenant en outre, dans une seconde période de détection :

la détection, par le premier détecteur, d'une troisième transmission par identification d'une troisième série d'impulsions espacées entre elles d'au moins un intervalle seuil entre impulsions ;

le masquage, par le second détecteur, de la troisième transmission ;

après masquage de la troisième transmission, la détection, par le second détecteur, d'une quatrième détection comme étant une transmission distincte de la troisième transmission, par identification d'une quatrième série d'impulsions espacées entre elles par l'au moins un intervalle seuil entre impulsions ;

l'attribution, par le hardware de traitement, d'un troisième intervalle entre impulsions pour la troisième transmission à un troisième bin de la pluralité de bins ; et

l'attribution, par le hardware de traitement, d'un quatrième intervalle entre impulsions pour la quatrième transmission à un quatrième bin de la pluralité de bins.

10. Le procédé de la revendication 9, dans lequel le premier, le second, le troisième, et le quatrième bin sont le même bin, et comprenant en outre :
la détermination, par le hardware de traitement, si la troisième ou la quatrième transmission correspond à une troisième capsule ingestible (14a-14c), en réponse à la détermination d'au moins l'un d'entre :

un troisième numéro de série pour la troisième transmission, ou un quatrième numéro de série pour la quatrième transmission, qui sont différents du premier et du second numéro de série, ou

une combinaison du troisième et du quatrième numéro de série qui est différente d'une combinaison du premier et du second numéro de série.

11. Le procédé de la revendication 5, dans lequel la première et la seconde transmission sont identifiées comme correspondant à au moins deux capsules ingestibles (14a-14c) en réponse à la détermination que le premier et le second numéro de série sont différents.

12. Le procédé de la revendication 5, dans lequel :

la réception de la première transmission comprend en outre la réception, par le hardware de traitement, d'une indication d'un premier niveau de batterie de la première capsule ingestible (14a-14c), et
la réception de la seconde transmission comprend en outre la réception, par le hardware de traitement, d'une indication d'un second niveau de batterie de la seconde capsule ingestible (14a-14c),
dans lequel la première et la seconde transmission sont identifiées comme correspondant à au moins deux capsules ingestibles (14a-14c) sur la base en outre du premier et du second niveau de batterie.

13. Un système d'étiquette biotélémétrique ingestible, comprenant :

une capsule (14a-14c) ;
un émetteur ; et
un hardware de traitement couplé à la capsule (14a-14c) et à l'émetteur, comprenant :
un composant électrique avec une valeur attendue de composant et une tolérance, une valeur réelle de composant pour le composant électrique ne variant pas dans le temps ; et
le hardware de traitement étant configuré pour :

générer une tension de sortie en fonction de la valeur réelle de composant pour le composant électrique ; et
générer un numéro de série aléatoire sur la base de la tension de sortie, le numéro de série aléatoire restant le même chaque fois que le système d'étiquette biotélémétrique ingestible est réinitialisé.

14. Le système d'étiquette biotélémétrique ingestible de la revendication 13, dans lequel le composant électrique est un premier composant électrique réalisé en un premier matériau, et le hardware de traitement comprend en outre:
un second composant électrique réalisé en un second matériau différent du premier matériau.

15. Le système d'étiquette biotélémétrique ingestible de la revendication 13, dans lequel le composant électrique est une résistance.

FIG. 1

FIG. 2

300

Pulse Space Distribution

FIG. 3

400

Battery Level

FIG. 4A

450

Unconditioned Burst Space

Mean: 6945.4 Sigma: 4.51

FIG. 4B

**FIG. 5**

FIG. 6

$$V_0 = Vref \left(1 - \frac{R2}{R1}\right)A + Vcm$$

EP 4 322 847 B1

700

| TXEND | SYNC | ID | SN | GI | TXSTART | 27 bits |
|-------|------|----|----|----|---------|---------|

Stored On IC — — Derived On IC

2    8    4    5    4    4

Last bit TXEND

First bit TXSTART

Bit 26

Bit 0

+T

**FIG. 7**

800

RF Signal → Receive and Demodulate to Baseband (scan over band) → Burst Detection → Message Correlate And Capsule ID Sort

Burst Pulse Stream

## FIG. 8A

850

Burst Pulse Strteam → Dual Presence Detect (DPD) → No → Message Correlation and Single Pulse Space with GI Qual (SME)

Yes

Dual Message Correlation

| Dual Pulse Space with GI Qual | Dual SN Extract |

(DME)

Px1  Px2    SNx1  SNx2

Pn

Multiple Transmission Detect and Sort (MTDS) → Capsule ID List

## FIG. 8B

**FIG. 9**

EP 4 322 847 B1

Capsule 2 Bursts

T2

1,2

T1

Bit 0     Capsule 1 Bursts     Bit 27

~ 178mS

**FIG. 10A**

1002

Capsule 1 Bursts

1

1004

Capsule 2 Bursts

2

**FIG. 10B**

EP 4 322 847 B1

1100

FIG. 11A

1150

FIG. 11B

EP 4 322 847 B1

1200

$$M = \begin{bmatrix} 2 \\ 3 \\ 4 \\ 5 \\ 6 \\ 7 \\ 8 \\ 9 \\ 10 \end{bmatrix} \quad PDA1\,(n,M) = \begin{bmatrix} 95.429 \\ 97.891 \\ 98.792 \\ 99.218 \\ 99.453. \\ 99.596 \\ 99.69 \\ 99.754 \\ 99.8 \end{bmatrix}$$

PDA(n,*M*) vs M
n=5 ingestions

**FIG. 12**

FIG. 13

1400

```
        ┌─────────────┐
        │    START    │
        └─────────────┘
               │
               ▼
┌──────────────────────────────┐
│     OBTAIN SERIAL NUMBER     │──── 1402
└──────────────────────────────┘
               │
               ▼
┌──────────────────────────────┐
│     MEASURE BATTERY LEVEL    │──── 1404
└──────────────────────────────┘
               │
               ▼
┌──────────────────────────────┐
│ GENERATE PULSE AMPLITUDE     │
│ MODULATED SIGNAL HAVING A    │──── 1406
│ PARTICULAR PULSE SPACE       │
│ AND INCLUDING SERIAL NUMBER  │
│ FIELD AND BATTERY LEVEL FIELD│
└──────────────────────────────┘
               │
               ▼
┌──────────────────────────────┐
│  TRANSMIT SIGNAL TO RECEIVER │──── 1408
└──────────────────────────────┘
               │
               ▼
        ┌─────────────┐
        │     END     │
        └─────────────┘
```

## FIG. 14

1500

```
         ┌─────────────┐
         │    START    │
         └─────────────┘
                │
                ▼
┌──────────────────────────────────┐
│   RECEIVE FIRST TRANSMISSION      │── 1502
│   HAVING FIRST PULSE SPACE AND    │
│   INCLUDING FIRST SERIAL NUMBER   │
│   AND FIRST BATTERY LEVEL         │
└──────────────────────────────────┘
                │
                ▼
┌──────────────────────────────────┐
│   RECEIVE SECOND TRANSMISSION     │── 1504
│   HAVING SECOND PULSE SPACE AND   │
│   INCLUDING SECOND SERIAL NUMBER  │
│   AND SECOND BATTERY LEVEL        │
└──────────────────────────────────┘
                │
                ▼
┌──────────────────────────────────┐
│   IDENTIFY FIRST AND SECOND       │── 1506
│   TRANSMISSIONS AS CORRESPONDING  │
│   TO TWO INGESTIBLE CAPSULES      │
│   BASED ON FIRST AND SECOND PULSE │
│   SPACES, FIRST AND SECOND        │
│   BATTERY LEVELS, AND/OR FIRST    │
│   AND SECOND SERIAL NUMBERS       │
└──────────────────────────────────┘
                │
                ▼
         ┌─────────────┐
         │     END     │
         └─────────────┘
```

**FIG. 15**

**REFERENCES CITED IN THE DESCRIPTION**

**Patent documents cited in the description**

- US 63174896

- US 2017258362 A1